# FASCICULE DE BREVET EUROPEEN

(11) **EP 2 144 570 B1**
(45) Date de publication et mention de la délivrance du brevet: **14.12.2011**
(21) Numéro de dépôt: 08805748.4
(22) Date de dépôt: 06.05.2008
(51) Int. Cl.: A61B 18/04, A61M 5/44

(54) **INSTALLATION DESTINEE A L'INJECTION DE VAPEUR D'EAU DANS UN VAISSEAU SANGUIN HUMAIN OU ANIMAL**
VORRICHTUNG ZUR INJEKTION VON WASSERDAMPF IN EIN MENSCHLICHES ODER TIERISCHES BLUTGEFÄSS
EQUIPMENT FOR INJECTION OF WATER VAPOUR INTO A HUMAN OR ANIMAL BLOOD VESSEL

(30) Priorité: 10.05.2007 FR 0754985
(43) Date de publication de la demande: 20.01.2010
(73) Titulaire: CERMAVEIN, 74160 Archamps (FR)
(72) Inventeur: MEHIER, Henri, F-69002 Lyon (FR)
(74) Mandataire: Fleurance, Raphaël
(86) Numéro de dépôt international: PCT/FR2008/050798
(87) Numéro de publication internationale: WO 2008/148996

(56) Documents cités:
- WO-A-00/29055
- WO-A-03/070302
- WO-A-2005/077290
- WO-A-2006/108974
- US-A- 5 456 662
- US-A1- 2006 122 590

## Description

L'invention a pour objet un nouveau dispositif permettant le traitement des pathologies veineuses ou artério-veineuse, en particulier des varices, des hémorroïdes et des shunts artério-veineux ou autre malformations vasculaires par injection de vapeur d'eau par voie endoluminale.

Plus précisément, l'invention a pour objet une installation destinée à l'injection en régime pulsé, de vapeur dans une veine humaine ou animale.

Le document US 2003/0109869 A1 décrit une méthode de traitement des varices. Le système proposé consiste à injecter de la vapeur directement dans la veine, de manière à détruire les veines variqueuses par chauffage du collagène présent sur leur paroi. En pratique, la transformation de l'eau en vapeur est effectuée directement dans la veine, l'eau étant chauffée au niveau de l'extrémité distale d'un cathéter par un arc électrique formé au moyen d'une électrode enroulée en spirale.

Cette installation présente plusieurs inconvénients.

Le premier concerne la puissance énergétique élevée pour transformer l'eau en vapeur par l'arc électrique. En outre et surtout, les cathéters constituent des produits consommables et donc non réutilisables d'un patient à l'autre ce qui, compte tenu de la complexité technique de ce type de cathéter, augmente considérablement le coût du matériel et donc de l'intervention. Pour diminuer ce coût, il serait intéressant de parvenir à transformer l'eau en vapeur non plus au niveau du cathéter consommable, mais plus en amont, c'est-à-dire au niveau du moyen d'amenée du liquide jusqu'au cathéter introduit dans la veine.

Dans un autre domaine qui concerne celui des ablations ou des cautérisations tissulaires, le document WO 02/069821 décrit un appareillage dans lequel la transformation de l'eau en vapeur est effectuée dans une pièce à main à l'extérieur de l'organisme. Dans le système proposé, la pièce à main abrite un tube présentant deux électrodes connectées à un générateur de radio-fréquence, le liquide assurant le passage du courant entre les deux électrodes. L'inconvénient de ce système est de disposer d'un tube dépourvu de chauffage différentiel, ce qui signifie que le tube est chauffé sur toute sa longueur. Dès lors, non seulement on observe une déperdition de chaleur, mais en outre et surtout, l'utilisateur, compte tenu des températures de chauffage, peut être gêné au moment de la préhension de la pièce à main et donc de l'intervention. Ce phénomène est renforcé du fait du régime permanent de production et de transfert de vapeur qui occasionne des échauffements non seulement pour l'utilisateur mais également et surtout pour le patient.

Le document WO 2006/108974 du Demandeur décrit une installation permettant d'injecter de la vapeur en régime pulsé non pas dans une veine, mais directement dans les tissus, en particulier pour le traitement des tumeurs. Dans le dispositif proposé, le chauffage n'est pas effectué dans la pièce à main, c'est-à-dire à l'extérieur de l'organisme, mais directement dans le cathéter en contact avec le tissu. A la différence du document précédent, le chauffage est ici différentiel, c'est-à-dire que la transformation de l'eau en vapeur est effectuée exclusivement à l'extrémité distale du tube incorporé dans l'organisme. Si cette installation permet un traitement efficace des tumeurs, elle présente en revanche l'inconvénient majeur de rester relativement onéreuse, dans la mesure où la miniaturisation du système de chauffage au niveau des tubes en contact avec l'organisme, c'est-à-dire des éléments consommables, a un coût très élevé.

Enfin, le document WO 03/070302 divulgue le préambule de la revendication 1,

En d'autres termes, le problème que se propose de résoudre l'invention est de développer une installation pour le traitement, en particulier des varices et des hémorroïdes par injection de vapeur par voie endoluminale, dont le système de chauffage et donc de transformation de l'eau en vapeur soit déporté à l'extérieur de l'organisme.

Autrement dit, l'objectif de l'invention est de mettre au point un dispositif dans lequel le changement d'état du liquide soit effectué dans la pièce à main en respectant les deux contraintes que sont le confort de préhension par le chirurgien de la pièce à main et le chauffage efficace du vaisseau et notamment de la veine au niveau de la varice ou de l'hémorroïde à traiter sans brûlure pour le patient au niveau de la peau.

Pour ce faire, le Demandeur a réussi à développer une pièce à main dans laquelle la transformation de l'eau en vapeur est exclusivement effectuée à l'extrémité distale de la dite pièce à main dans une zone non en contact avec la main du chirurgien, l'ensemble des éléments de l'installation étant mis au point et agencés pour permettre l'administration de vapeur en régime pulsé, jusqu'à l'extrémité distale du dispositif, c'est-à-dire au niveau des vaisseaux à traiter, le régime pulsé permettant le transfert rapide des calories de la pièce à main à l'extrémité du moyen de diffusion de façon à apporter localement des calories le plus rapidement possible pour diminuer les pertes thermiques indésirables.

L'invention a donc pour objet une installation destinée à l'injection en régime pulsé de vapeur d'eau dans un vaisseau humain ou animal comprenant :
- une unité d'injection d'eau froide en régime pulsé dans une pièce à main,
- une pièce à main temporairement solidaire de l'unité d'injection au sein de laquelle est agencé un tube métallique dans lequel l'eau froide est transformée en vapeur,
- un moyen de diffusion de la vapeur dans le vaisseau destiné à être connecté de manière réversible à l'extrémité distale de la pièce à main,
caractérisée en ce que le tube métallique :
- a un diamètre externe compris entre 200 µm et 1 000 µm, avantageusement de l'ordre de 800 µm et un diamètre interne compris entre 100 µm et 500 µm, avantageusement de l'ordre de 250 µm,
- présente une extrémité distale enroulée sur elle-même pour former une spirale,
et en ce que les deux extrémités du tube sont connectées à une source électrique, la portion de tube comprise entre son extrémité proximale et l'extrémité proximale de la spirale étant gainée d'un matériau, avantageusement une tresse de cuivre, présentant une résistivité telle que seule la spirale est apte à chauffer à une température permettant le passage de l'eau de la phase liquide à la phase vapeur.

En d'autres termes, le Demandeur a réussi à mettre au point un système dans lequel le chauffage différentiel est rendu possible au niveau de la pièce à main, c'est-à-dire au sein d'une pièce semi-consommable, diminuant ainsi le coût de l'installation. En pratique, le semi-consommable peut en effet être réutilisable pour une vingtaine d'interventions. En outre, la conformation spécifique du microtube dans lequel circule l'eau au niveau de la pièce à main permet de maintenir l'état vapeur de l'extrémité distale de la pièce à main à l'extrémité distale du moyen de diffusion.

En pratique et selon une autre caractéristique, la spirale est formée autour d'une portion d'un tube métallique de faible résistance électrique, en pratique inférieure à 0.5 ohm, permettant d'assurer le retour du courant électrique sans échauffement, dont :
- l'extrémité proximale en contact avec la spirale est gainée d'une matière isolante électriquement et thermiquement, la partie conductrice du tube étant connectée à la source électrique amenant le courant,
- l'extrémité distale est dépourvue de gaine isolante électriquement et thermiquement et est en contact avec l'extrémité distale du tube parcouru par le liquide.

Par ailleurs, compte tenu du diamètre du tube parcouru par le liquide et de la taille de la pièce à main qui doit être facilement manipulable par le chirurgien, la longueur du tube enroulé pour former une spirale est comprise entre 10 et 30 cms, avantageusement de l'ordre de 20 cms.

Pour éviter les risques de court-circuit et diminuer au maximum la température de chauffage au niveau de l'extrémité distale de la pièce à main, l'extrémité distale du tube dans la zone dans laquelle est agencée la spirale est gainée par une couche isolante thermiquement et électriquement.

Avantageusement, pour améliorer encore l'isolation thermique, l'extrémité distale de la pièce à main est constituée au niveau de la zone dans laquelle est agencée la spirale, d'un matériau isolant thermiquement et en particulier de silicone.

Pour permettre au chirurgien de disposer d'une certaine liberté de mouvement, la pièce à main est avantageusement connectée de manière irréversible à une rallonge reliant l'unité d'injection d'eau froide. Dans cette configuration, l'installation comporte alors un équipement non consommable (unité d'injection), des éléments semi-consommables (pièce à main et rallonge) et des éléments consommables (moyen de diffusion).

S'agissant de ce dernier, il peut présenter trois formes distinctes.

Dans les deux premiers cas, le moyen de diffusion se présente sous la forme d'un tube métallique de diamètre interne et externe avantageusement inférieurs à ceux du tube localisé dans la pièce à main, compris entre 100 et 200 µm, avantageusement 150 µm pour le diamètre interne et entre 250 et 500 µm, avantageusement 350 µm pour le diamètre externe, permettant ainsi de lui conférer une certaine souplesse et de diminuer les échanges thermiques.

Dans un premier mode de réalisation, l'extrémité distale du microtube est obturée, avantageusement par le biais d'une pièce en acier inoxydable arrondie rapportée sur ladite extrémité, le tube présentant à proximité de son extrémité distale, soit sur le tube proprement dit, soit sur la pièce rapportée, au moins un orifice traversant. Pour éviter la nécrose de la paroi des vaisseaux, le tube est recouvert sur toute sa surface, mais à l'exception de l'éventuel orifice traversant, d'une couche d'un matériau isolant thermiquement tel que notamment PTFE, PEEK, polyimide ou silicone, avantageusement PTFE.

Dans un autre mode de réalisation, le microtube est gainé d'un matériau isolant thermiquement, la gaine n'étant pas fixe mais mobile le long du microtube. Dans ce cas de figure, la longueur de la gaine isolante est supérieure à la longueur du microtube, ce qui permet, en raison de la souplesse de la gaine, d'éviter de perforer le vaisseau avec le microtube au moment de l'introduction de ce dernier dans ledit vaisseau. En pratique, le microtube présente un orifice positionné à son extrémité terminale assurant la délivrance des pulses de vapeur.

Dans ces deux modes de réalisation, le microtube présente sur toute sa longueur un marquage, en pratique tous les centimètres, permettant d'indiquer à l'opérateur la longueur restante de microtube dans le vaisseau au fur et à mesure du retrait de celui-ci.

Dans un autre mode de réalisation, le moyen de diffusion ne se présente pas sous la forme d'un microtube, mais sous forme d'une aiguille, l'aiguille étant recouverte sur au moins sa surface en contact avec le vaisseau, d'un matériau isolant thermiquement,, tel que par exemple du PTFE. En pratique, l'aiguille présente trois portions obtenues par rectification de section externe décroissante de l'extrémité proximale vers l'extrémité distale, respectivement :
- une portion proximale de diamètre externe compris entre 1,4 et 1,9 mm,
- une portion médiane de diamètre externe compris entre 1 et 1,3 mm,
- une portion distale de diamètre externe compris entre 0,5 et 0,8 mm,
le diamètre interne de l'aiguille étant constant et compris entre 0,1 et 0,25 mm, avantageusement égal à 0,15 mm.

Bien entendu, moyen de diffusion et pièce à main sont connectés par tout moyen adéquat connu de l'homme du métier.

S'agissant de l'unité d'injection, celle-ci s'apparente à l'unité décrite dans le document WO 2006/108974 incorporé ici par référence. Plus particulièrement, l'unité d'injection se présente sous la forme d'une chambre contenant l'eau à injecter et dans laquelle un vérin hydraulique piloté par un vérin électrique, pneumatique, piézoélectrique, ou mécanique, dont le déclenchement et/ou la force et/ou la vitesse de déplacement sont déterminés en fonction du rythme, du volume et de la pression d'injection souhaitée de la substance dans la pièce à main par le vérin hydraulique. L'unité d'injection peut également être associée à une unité de stockage d'eau froide.

L'invention a également pour objet une méthode de traitement des pathologies veineuses ou artério-veineuses, en particulier des varices, des hémorroïdes et des shunts artério-veineux, et plus généralement des malformations vasculaires par injection de pulses de vapeur dans les vaisseaux (veines et/ou artères) par voie endoluminale au moyen de l'installation précédemment décrite. L'avantage de l'installation est de permettre la diffusion homogène de vapeur sur plusieurs centimères (en pratique 4 à 5 cm) dans les vaisseaux, ce qui évite tout risque de carbonisation ou de perforation de la paroi tel qu'il pourrait exister avec l'installation décrite précédemment dans le document US 2003/0109869 ou dans les autres techniques thermiques existantes, générant une chaleur ponctuelle.

L'invention et les avantages qui en découlent ressortiront mieux des exemples de réalisation suivants à l'appui des figures annexées.
La figure lest une représentation schématique de l'installation de l'invention.
La figure 2 est une représentation en coupe de la pièce à main et de la rallonge de l'invention.
La figure 3 est une représentation en détail de l'extrémité distale de la pièce à main, objet de la figure 2.
La figure 4 est une illustration schématique du moyen de diffusion dans un premier mode de réalisation.
Les figures 5 et 6 sont des représentations schématiques du moyen de diffusion dans un second mode de réalisation.
La figure 7 est une représentation schématique du moyen de diffusion dans un troisième mode de réalisation.

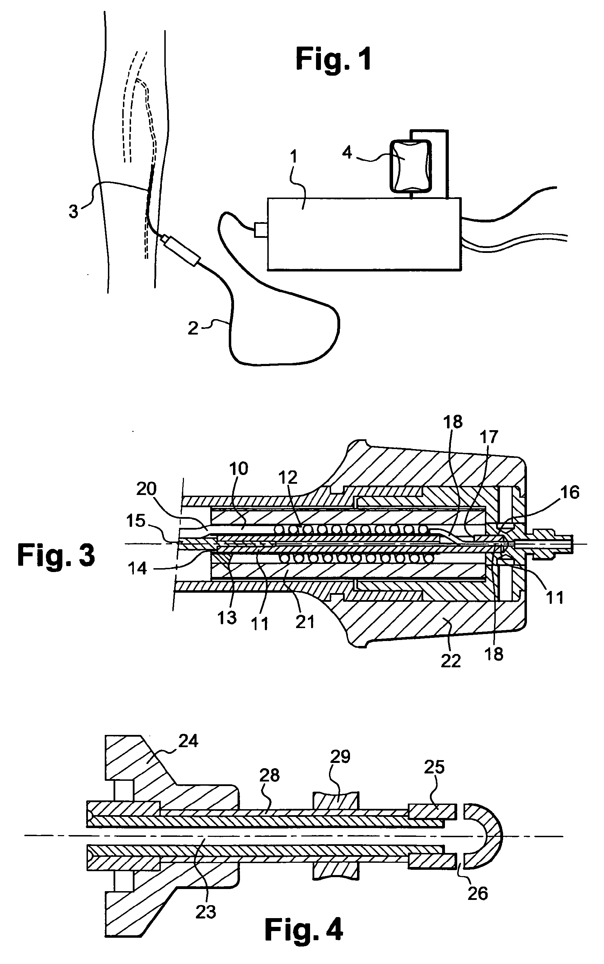

La figure 1 représente l'installation de l'invention de manière schématique. Comme le montre cette figure, l'installation comprend trois éléments essentiels que sont :
- une unité d'injection d'eau froide (1) sous forme de pulses,
- un ensemble pièce à main-rallonge (2) à l'extrémité distale duquel l'eau est transformée en vapeur,
- un moyen de diffusion de la vapeur d'eau dans la veine sous la forme d'un cathéter ou microtube (3).

En pratique, l'unité d'injection est en outre associée à une poche (4) de stockage d'eau.

Le générateur de pulses d'eau froide ne nécessite pas d'être décrit dans le détail et s'apparente à celui illustré dans la demande précitée WO 2006/108974 A1. En pratique, l'unité d'injection se présente sous forme d'une chambre contenant une substance à injecter, en l'espèce de l'eau froide, dans laquelle un vérin hydraulique de faible diamètre, de l'ordre de 3 à 5 mm, est piloté par un vérin électrique, pneumatique, piézoélectrique ou mécanique de diamètre plus important, de l'ordre de 50 à 80 mm, dont le déclenchement et/ou la course et/ou la force et/ou la vitesse de déplacement sont déterminés en fonction du rythme, du volume et de la pression d'injection souhaités, de la substance dans l'ensemble rallonge-pièce à main par le vérin hydraulique.

Pour éviter le retour de l'eau dans l'unité d'injection après injection de ladite eau dans l'ensemble pièce à main-rallonge, l'unité d'injection contient deux clapets anti-retour (non représentés). Comme déjà dit, la pression à laquelle la substance est injectée dépend de la vitesse de déplacement et de la force des vérins, par exemple pneumatiques, qui sont également programmés.

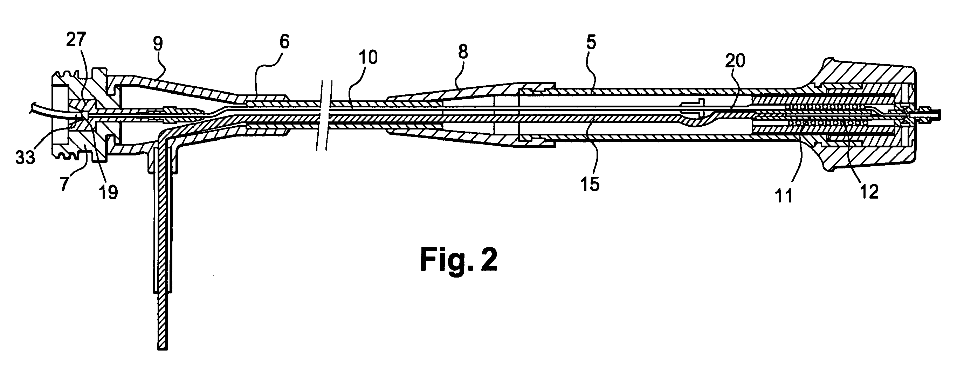

Le moyen de diffusion de la vapeur d'eau dans le vaisseau est séparé de l'unité d'injection par un ensemble pièce à main-rallonge (2) représenté sur la figure 2. Plus précisément, l'ensemble illustré comprend une pièce à main (5), une rallonge (6), ainsi qu'un moyen de connexion (7) à l'unité d'injection sous forme d'un pas de vis. La pièce à main (5), ainsi que la rallonge (6) ont une forme générale tubulaire. La connexion entre la rallonge et la pièce à main d'une part, et le pas de vis (7) d'autre part, est obtenue par la mise en place des pièces intermédiaires (8, 9). Les pièces (8, 9) sont montées de manière irréversible. L'ensemble rallonge-pièce à main constitue ainsi un élément semi-consommable. En pratique, la rallonge peut atteindre 1,5 à 5 m de longueur tandis que la pièce à main a une taille d'environ 10 à 20 cms.

Selon l'invention, l'ensemble rallonge-pièce à main est parcouru par un tube (10) dans lequel circule le liquide. Le tube est réalisé en inox et a un diamètre interne égal à 250 µm et un diamètre externe égal à 800 µm. Le matériau constitutif du tube a une résistivité égale à 72 µΩ/cm.

Comme le montre la figure 3, l'extrémité distale du tube (10) est enroulée autour d'une portion de tube en inox (11) de faible résistance pour former une spirale (12), en pratique de 20 cms linéaire. La portion de tube en inox (11) en contact avec la spirale (12) est recouverte d'une gaine isolante électriquement et thermiquement (13). L'extrémité proximale (14) de la portion de tube (11) est en contact avec un conducteur électrique (15) connecté au pôle positif d'une source électrique non représentée. L'extrémité distale (16) de la portion de tube (11) présente quant à elle une ouverture (17) permettant le passage de l'extrémité distale (18) de la spirale (12) de manière à être en contact avec le conducteur électrique formé par la portion de tube inox (11) de faible résistance, en pratique inférieure à 0.5 Ω. Comme le montre la figure 3, l'extrémité distale de la portion de tube (11) est dépourvue de gaine isolante permettant ainsi le passage du courant du tube inox (11) dans la spirale (12). Pour permettre le chauffage dans la seule portion en spirale du tube (10) et non de sa partie rectiligne présente entre son extrémité proximale (19) à la sortie de l'unité d'injection et l'extrémité proximale (20) de la spirale (12), la partie rectiligne est munie d'une gaine sous la forme d'une tresse de cuivre de faible résistivité. Le pas de vis (7) intègre la connexion hydraulique qui dans le même temps fait office de masse électrique (27). En d'autres ternes, le courant circule dans le conducteur électrique (15), puis dans la spirale (12) pour revenir par le tube (10) jusqu'à la masse (27).

Selon une autre caractéristique, la spirale (12) est munie d'une gaine isolante électriquement et thermiquement (21). Par ailleurs, l'extrémité distale de la pièce à main est munie d'une pièce en silicone (22) permettant d'éviter un échauffement trop important de la pièce à main.

La figure 4 est une représentation d'un moyen de diffusion dans un premier mode de réalisation. Dans cette configuration, le moyen de diffusion se présente sous la forme d'un microtube (23) de diamètre interne égal à 150 µm et de diamètre externe égal à 350 µm. Ce microtube (23) est connecté à l'extrémité distale de la pièce à main par tout moyen adéquat défini schématiquement par la référence 24. Selon une caractéristique essentielle, l'extrémité distale du microtube est obturée par une pièce inox (25) rapportée munie d'un orifice traversant (26) permettant le passage de la vapeur. En pratique, à l'exception de la pièce rapportée, le microtube est recouvert d'une substance thermiquement isolante du type PTFE et/ou PEEK (28). Le microtube présente en outre à proximité de son extrémité proximale, un moyen de préhension (29) facilitant la mise en place par le chirurgien du microtube dans le vaisseau.

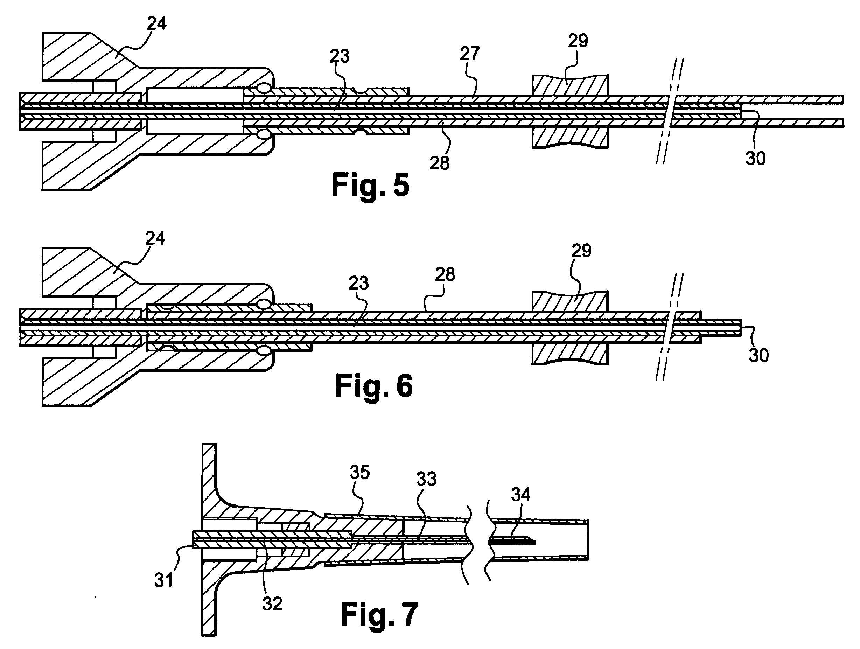

Dans les modes de réalisation objets des figures 5 et 6, la gaine de PTFE (28), agencée autour du microtube (23) n'est plus fixe, mais mobile. Dans cette hypothèse, le microtube n'est pas obturé et donc muni d'un orifice terminal (30).

La figure 5 représente le moyen de diffusion au moment de sa mise en place, alors que la figure 6 représente le même moyen de diffusion au moment du traitement.

Comme le montrent ces figures, la gaine ou tube PTFE (28) a une longueur supérieure à celle du microtube de manière à éviter toute perforation dans la zone à traiter par ledit microtube au moment de sa mise en place. En position de traitement, le tube PTFE est retiré vers l'arrière par le biais de la pièce (29) restée à l'extérieur de l'organisme. Bien entendu, la connexion entre l'extrémité proximale du microtube et l'extrémité distale de la pièce à main est effectuée par tout moyen connu, représenté schématiquement par la référence 24.

La figure 7 est une représentation du moyen de diffusion dans un troisième mode de réalisation. Dans ce cas, le microtube est remplacé par une aiguille (31) de diamètre interne continu égal à 0,15 mm présentant trois portions distinctes obtenues par rectification, respectivement :
- une portion proximale (32) de diamètre externe égal à 1,6 mm,
- une portion médiane (33) de diamètre externe égal à 1,2 mm, et
- une extrémité distale (34) de diamètre externe égal à 0,7 mm.

La partie de l'aiguille destinée à être introduite dans le vaisseau est enduite d'une gaine de téflon ou dépôt silicone non représentée. Par ailleurs, pendant son stockage, l'aiguille est entourée d'un tube de protection (35) d'une longueur sensiblement supérieure à celle de l'aiguille.

Comme déjà dit, l'appareil de l'invention est destiné au traitement des pathologies veineuses ou artério-veineuses et plus particulièrement au traitement des varices ou des hémorroïdes. En pratique, le générateur est conformé pour délivrer des pulses d'eau de volume compris entre 50 et 100 µl, avantageusement 70 µl, permettant de véhiculer entre 30 et 100 J, avantageusement de l'ordre de 50 J.

La méthode de traitement va maintenant être décrite dans le détail en relation avec les varices et la mise en oeuvre d'un moyen de diffusion sous forme d'un microtube (figures 4 à 6).

On procède au préalable à l'évaluation diagnostic de la pathologie par échographie Doppler. On effectue alors un repérage de la veine à traiter par marquage sur la peau du cheminement de la veine et de l'orifice d'introduction du microtube délivrant la vapeur.

En fonction du diamètre du vaisseau à traiter, on détermine alors le nombre de pulses à délivrer par centimètre linéaire de vaisseau à traiter. A titre d'exemple, pour une veine de 12 mm de diamètre, on envoie deux pulses, chacun de 70 µl d'eau, chaque pulse véhiculant une énergie de 50 J.

Le traitement peut alors débuter soit sous anesthésie locale, soit sous anesthésie générale en fonction du souhait du patient.

La méthode de traitement consiste tout d'abord à ponctionner le vaisseau, et plus particulièrement la veine à traiter, au moyen d'une aiguille positionnée dans un petit cathéter d'environ 5 cm de longueur dont la surface est téflonnée, l'aiguille étant retirée après mise en place du cathéter à la surface de la peau. On introduit ensuite dans le cathéter, le microtube dans l'une des configurations des figures 4 ou 5, 6 précédentes, jusqu'à ce que l'extrémité distale du microtube atteigne l'extrémité de la veine à traiter.

Le générateur envoie alors les pulses d'eau froide dans la pièce à main, laquelle transforme ces pulses en vapeur à une température d'environ 200°C, la vapeur étant ensuite véhiculée par le microtube jusqu'à son extrémité distale.

Grâce au marquage réalisé à la surface du microtube, l'opérateur retire progressivement ledit microtube au rythme de 1 à plusieurs pulses par centimètres, en fonction du diamètre du vaisseau. Selon une caractéristique essentielle, il n'est pas nécessaire que le retrait soit continu et régulier, ce qui fait que l'installation ne requiert pas un appareil additionnel permettant d'automatiser le retrait.

L'installation peut également être appliquée au traitement des hémorroïdes.

Dans ce cas, on visualise les lésions par le biais d'un anuscope. On met ensuite en place des forceps à la base de l'hémorroïde de manière à interrompre le flux sanguin et limiter le transfert de chaleur au niveau de la paroi anale. On introduit ensuite une fine aiguille de type de celle précédemment décrite (figure 7), entourée d'une matière isolante pour protéger la muqueuse contre les brûlures, le tout sous contrôle visuel. On débute alors le chauffage et on émet 1 à 3 pulses de vapeur. L'aiguille est ensuite retirée, les forceps ouverts et également retirés. Pendant le traitement, on peut effectuer un refroidissement par air ou par liquide, de manière à protéger les structures environnantes. On peut aussi protéger la muqueuse anale par un gel éventuellement anesthésiant.

L'invention et les avantages qui en découlent ressortiront bien de la description qui précède. On note en particulier la mise au point d'une installation apte à injecter des pulses de vapeur directement dans une veine et où le chauffage est effectué dans des semi-consommables et non des consommables.

En outre, un des points intéressants de la technique mise au point par le Demandeur est de disposer d'une température homogène sur 5 à 6 cm de longueur de veine, ce qui permet de retirer par pas successif, le moyen de diffusion. Au contraire, les autres technologies, telles que RF ou laser procurent une chaleur ponctuelle avec le risque de nécroser ponctuellement toute la paroi de la veine.

Un autre avantage de la technique est de pouvoir traiter indifféremment une veine avec son sang ou vidée de son sang, alors que le laser traite la veine avec son sang et la RF traite la veine vidée de son sang.

## Revendications

1. Installation destinée à l'injection en régime pulsé de vapeur d'eau dans un vaisseau humain ou animal comprenant :
- une unité d'injection (1) d'eau froide en régime pulsé dans une pièce à main (5),
- une pièce à main (5) temporairement solidaire de l'unité d'injection (1) au sein de laquelle est agencé un tube métallique (10) dans lequel l'eau froide est transformée en vapeur,
- un moyen de diffusion de la vapeur dans le vaisseau destiné à être connecté de manière réversible à l'extrémité distale de la pièce à main,
dans la quelle le tube métallique (10) a
- un diamètre interne compris entre 100 hum et 500 µm,
- présente une extrémité distale enroulée sur elle-même pour former une spirale (12),
et **caracterisée en ce que** le tube métallique a un diamètre externe compris entre 200 µm et 1 000 µm et les deux extrémités du tube sont connectées à une source électrique (27, 15),
la portion de tube comprise entre son extrémité proximale (19 et l'extrémité proximale (20) de la spirale (12) étant gainée d'un matériau présentant une résistivité telle que seule la spirale chauffe à une température permettant le passage de l'eau de la phase liquide à la phase vapeur.

2. Installation selon la revendication 1, **caractérisée en ce que** la spirale (12) est formée autour d'une portion d'un tube métallique (11) de faible résistance électrique dont :
- l'extrémité proximale 14) en contact avec la spirale (12) est gainée d'une matière isolante électriquement et thermiquement (13), la partie conductrice du tube étant connectée à la source électrique amenant le courant,
- l'extrémité distale (16) est dépourvue de gaine isolante électriquement et thermiquement et est en contact avec l'extrémité distale du tube (10) parcouru par le liquide.

3. Installation selon l'une des revendications précédentes, **caractérisé en ce que** la longueur de tube enroulé pour former une spirale (12) est comprise entre 10 et 30 cms, avantageusement voisine de 20 cms.

4. Installation selon l'une des revendications précédentes, **caractérisé en ce que** l'extrémité distale du tube (10) dans la zone dans laquelle est agencée la spirale (12) est gainée par une couche isolante (21) thermiquement et électriquement.

5. Installation selon l'une des revendications précédentes, **caractérisé en ce que** l'extrémité distale de la pièce à main est constituée, au niveau de la zone dans laquelle est agencée la spirale, d'un matériau isolant thermiquement, en particulier du silicone (22).

6. Installation selon l'une des revendications précédentes, **caractérisée en ce que** la pièce à main (5) est rigide et connectée de manière irréversible à une rallonge souple (9).

7. Installation selon l'une des revendications précédentes, **caractérisée en ce que** le moyen de diffusion se présente sous la forme d'un tube métallique (23) de diamètre interne compris entre 100 et 200 µm et externe compris entre 250 et 500 µm, dont l'extrémité distale est obturée par le biais d'une pièce (25) rapportée en acier munie d'au moins un orifice traversant (26), le tube étant par ailleurs recouvert, sur toute sa surface, d'une couche d'un matériau isolant thermiquement (28).

8. Installation selon l'une des revendications 1 à 6, **caractérisée en ce que** le moyen de diffusion se présente sous la forme d'un tube métallique (23) de diamètre interne compris entre 100 et 200 µm et externe compris entre 250 et 500 µm, dont l'extrémité distale est ouverte (30), le tube étant recouvert d'une gaine isolante (28) thermiquement, mobile le long du tube et de taille supérieure à celle du tube métallique.

9. Installation selon l'une des revendications 1 à 6, **caractérisée en ce que** le moyen de diffusion se présente sous la forme d'une aiguille (31) recouverte sur au moins la surface destinée à être en contact avec un matériau isolant thermiquement, l'aiguille présentant trois portions obtenues par rectification de section externe décroissante de l'extrémité proximale vers l'extrémité distale, respectivement :
- une portion proximale (32) de diamètre externe compris entre 1,4 et 1,9 mm,
- une portion médiane (33) de diamètre externe compris entre 1 et 1,3 mm,
- une portion distale (34) de diamètre externe compris entre 0,5 et 0,8 mm,
le diamètre interne de l'aiguille étant constant et compris entre 0.1 et 0.25 mm, avantageusement égal à 0.15 mm.

10. Installation selon l'une des revendications précédentes, **caractérisée en ce que** l'unité d'injection (1) se présente sous la forme d'une chambre contenant l'eau à injecter et dans laquelle un vérin hydraulique piloté par un vérin électrique, pneumatique, piézoélectrique ou mécanique, dont le déclenchement et/ou la force et/ou la vitesse de déplacement sont déterminés en fonction du rythme, du volume et de la pression d'injection souhaitée de la substance dans la pièce à main par le vérin hydraulique.

## Claims

1. Apparatus for injecting pulsed steam into a human or animal vessel comprising:
- an injection unit (1) sending pulses of cold water into a handpiece (5),
- a handpiece (5) temporarily attached to the injection unit (1) within which is a metal tube (10) in which the cold water is transformed into steam,
- a means of distributing the steam to the vessel intended to be connected in a reversible manner to the distal end of the handpiece,
wherein the metal tube (10) has an internal diameter between 100 µm and 500 µm, and has a distal end coiled to form a spiral (12),
and **characterized in that** the metal tube as an external diameter between 200 µm and 1,000 µm, and the two extremities of the tube are connected to an electric supply (27, 15), the portion of the tube between its proximal end (19) and the proximal end (20) of the spiral (12) being sheathed with a material of resistivity such that only the spiral heats to a temperature allowing the water to change from the liquid to the vapor phase.

2. Apparatus according to claim 1, **characterized in that** the spiral (12) is formed around a portion of a metal tube (11) of low electrical resistance of which:
- the proximal end (14) in contact with the spiral (12) is sheathed with an electrical and thermal insulating material (13), the conducting part of the tube being connected to the electrical supply providing the current,
- the distal end (16) has no electrical and thermal insulating sheath and is in contact with the distal end of the tube (10) through which the liquid runs

3. Apparatus according to one of the preceding claims, **characterized in that** the length of tube rolled up to form a spiral (12) is between 10 and 30 cm, advantageously close to 20 cm.

4. Apparatus according to one of the preceding claims, **characterized in that** the distal end of the tube (10) in the area where the spiral (12) is located is sheathed in an electrical and thermal insulating layer (21).

5. Apparatus according to one of the preceding claims, **characterized in that** the distal end of the handpiece where the spiral is located is composed of a thermal insulating material, in particular silicone (22).

6. Apparatus according to one of the preceding claims, **characterized in that** the handpiece (5) is rigid and connected to a flexible extension (9) in such a way that it cannot be disassembled.

7. Apparatus according to one of the preceding claims, **characterized in that** the means of distribution is in the form of a metal tube (23) of internal diameter between 100 and 200 µm and external diameter between 250 and 500 µm, the distal end of which is closed by an additional steel part (25) having at least one transverse opening (26), the whole surface of the tube being in addition covered with a layer of thermal insulating material (28).

8. Apparatus according to one of claims 1 to 6, **characterized in that** the means of distribution is in the form of a metal tube (23) of internal diameter between 100 and 200 µm and external diameter between 250 and 500 µm, the distal end of which is open (30), the tube being covered by a thermal insulating sheath (28) which can move along the tube and which is longer than the metal tube.

9. Apparatus according to one of claims 1 to 6, **characterized in that** the means of distribution is in the form of a needle (31) covered with a thermal insulating material at least over the area intended to be in contact, this needle having three sections decreasing in size obtained by grinding the external cross-section from the proximal end to the distal end, and respectively:
- a proximal portion (32) with external diameter between 1.4 and 1.9 mm,
- a middle portion (33) with external diameter between 1 and 1.3 mm,
- a distal portion (34) with external diameter between 0.5 and 0.8 mm,
the internal diameter of the needle being constant and between 0.1 and 0.25 mm, to advantage equal to 0.15 mm.

10. Apparatus according to one of the preceding claims, **characterized in that** the injection unit (1) is in the form of a chamber containing the water to be injected, in which there is a hydraulic piston driven by an electric, pneumatic, piezoelectric or mechanical piston, the activation and/or force and/or speed of movement of which are determined by the hydraulic piston according to the rhythm, volume and pressure required for injection of the substance into the handpiece.

## Patentansprüche

1. Anlage, die dazu bestimmt ist, Wasserdampf im gepulsten Betrieb in ein menschliches oder tierisches Gefäß einzuleiten, die Folgendes umfasst:
- eine Einleitungseinheit (1) zum Einleiten von kaltem Wasser im gepulsten Betrieb in ein Handteil (5),
- ein Handteil (5), das mit der Einleitungseinheit (1) vorübergehend fest verbunden ist und in dem ein Metallrohr (10) angeordnet ist, in dem das kalte Wasser in Dampf umgewandelt wird,
- ein Diffusionsmittel für den Dampf in dem Gefäß, das dazu bestimmt ist, mit dem distalen Ende des Handteils lösbar verbunden zu werden,
- wobei das Metallrohr (10) einen Innendurchmesser besitzt, der im Bereich von 100 µm bis 500 µm liegt, und
- ein distales Ende aufweist, das auf sich selbst gewickelt ist, um eine Spirale (12) zu bilden,
und **dadurch gekennzeichnet, dass** das Metallrohr einen Außendurchmesser im Bereich von 200 µm bis 1000 µm aufweist und die zwei Enden des Rohrs mit einer Stromquelle (27, 15) verbunden sind, wobei der Abschnitt des Rohrs zwischen seinem proximalen Ende (19) und dem proximalen Ende (20) der Spirale (12) mit einem Material abgeschirmt ist, das einen spezifischen elektrischen Widerstand aufweist, derart, dass nur die Spirale auf eine Temperatur erwärmt wird, die den Übergang des Wassers von der flüssigen Phase in die Dampfphase ermöglicht.

2. Anlage nach Anspruch 1, **dadurch gekennzeichnet, dass** die Spirale (12) um einen Abschnitt eines Metallrohrs (11) mit geringem elektrischen Widerstand ausgebildet ist, wovon:
- das proximale Ende (14), das mit der Spirale (12) in Kontakt ist, mit einem elektrisch und thermisch isolierenden Material (13) abgeschirmt ist, wobei der leitende Teil des Rohrs mit der Stromquelle, die Strom liefert, verbunden ist,
- das distale Ende (16) keine elektrisch und thermisch isolierende Abschirmung aufweist und mit dem distalen Ende des Rohrs (10), durch das die Flüssigkeit strömt, in Kontakt ist.

3. Anlage nach einem der vorhergehenden Ansprüche, **dadurch gekennzeichnet, dass** die Länge des Rohrs, das aufgewickelt ist, um eine Spirale (12) zu bilden, im Bereich von 10 bis 30 cm liegt und vorzugsweise in der Umgebung von 20 cm liegt.

4. Anlage nach einem der vorhergehenden Ansprüche, **dadurch gekennzeichnet, dass** das distale Ende des Rohrs (10) in der Zone, in der die Spirale (12) angeordnet ist, durch eine thermisch und elektrisch isolierende Schicht (21) abgeschirmt ist.

5. Anlage nach einem der vorhergehenden Ansprüche, **dadurch gekennzeichnet, dass** das distale Ende des Handteils auf Höhe der Zone, in der die Spirale angeordnet ist, aus einem thermisch isolierenden Material, insbesondere Silikon (22), gebildet ist.

6. Anlage nach einem der vorhergehenden Ansprüche, **dadurch gekennzeichnet, dass** das Handteil (5) starr ist und mit einer nachgiebigen Verlängerung (9) irreversibel verbunden ist.

7. Anlage nach einem der vorhergehenden Ansprüche, **dadurch gekennzeichnet, dass** das Diffusionsmittel die Form eines Metallrohrs (23) mit einem Innendurchmesser im Bereich von 100 bis 200 µm und einem Außendurchmesser im Bereich von 250 bis 500 µm aufweist, wovon das distale Ende durch ein eingesetztes Stahlteil (25) verschlossen ist, das mit wenigstens einer Durchgangsöffnung (26) versehen ist, wobei das Rohr außerdem auf seiner gesamten Oberfläche mit einer Schicht aus einem thermisch isolierenden Material (28) abgedeckt ist.

8. Anlage nach einem der Ansprüche 1 bis 6, **dadurch gekennzeichnet, dass** das Diffusionsmittel die Form eines Metallrohrs (23) mit einem Innendurchmesser im Bereich von 100 bis 200 µm und einem Außendurchmesser im Bereich von 250 bis 500 µm aufweist, wovon das distale Ende offen (30) ist, wobei das Rohr mit einer thermisch isolierenden Abschirmung (28) abgedeckt ist, die längs des Rohrs beweglich ist und die eine Größe hat, die größer als jene des Metallrohrs ist.

9. Anlage nach einem der Ansprüche 1 bis 6, **dadurch gekennzeichnet, dass** das Diffusionsmittel die Form einer Nadel (31) aufweist, die wenigstens auf der Oberfläche, die dazu bestimmt ist, mit einem thermisch isolierenden Material in Kontakt zu sein, abgedeckt ist, wobei die Nadel drei Abschnitte aufweist, die durch Begradigung erhalten werden und einen vom proximalen Ende zum distalen Ende abnehmenden Außenquerschnitt aufweisen, nämlich:
- einen proximalen Abschnitt (32) mit einem Außendurchmesser im Bereich von 1,4 bis 1,9 mm,
- einen mittleren Abschnitt (33) mit einem Außendurchmesser im Bereich von 1 bis 1,3 mm,
- einen distalen Abschnitt (34) mit einem Außendurchmesser im Bereich von 0,5 bis 0,8 mm,
wobei der Innendurchmesser der Nadel konstant ist und im Bereich von 0,1 bis 0,25 mm liegt und vorteilhaft gleich 0,15 mm ist.

10. Anlage nach einem der vorhergehenden Ansprüche, **dadurch gekennzeichnet, dass** die Einleitungseinheit (1) die Form einer Kammer aufweist, die einzuleitendes Wasser enthält und in der ein Hydraulikzylinder durch einen elektrischen, pneumatischen, piezoelektrischen oder mechanischen Zylinder vorgesteuert wird, dessen Auslösung und/oder Kraft und/oder Verlagerungsgeschwindigkeit als Funktion des Takts, des Volumens und des Drucks des gewünschten Einleitens der Substanz in das Handteil durch den Hydraulikzylinder bestimmt sind.
